**Europäisches Patentamt**

**European Patent Office**

**Office européen des brevets**

(19)

(11) Veröffentlichungsnummer: **0 231 885**
**B1**

(12)

# EUROPÄISCHE PATENTSCHRIFT

(45) Veröffentlichungstag der Patentschrift:
06.03.89

(51) Int. Cl.⁴: **A 61 B 17/56**, A 61 B 19/00

(21) Anmeldenummer: **87101223.3**

(22) Anmeldetag: **29.01.87**

(54) **Passer für Schienbeinoperationen, die zur Korrigierung von fehlerhaften Stellungen des Knies ausgeführt werden.**

(30) Priorität: **30.01.86 FI 860442**

(43) Veröffentlichungstag der Anmeldung:
**12.08.87 Patentblatt 87/33**

(45) Bekanntmachung des Hinweises auf die Patenterteilung:
**06.03.89 Patentblatt 89/10**

(84) Benannte Vertragsstaaten:
**AT BE CH DE ES FR GB GR IT LI LU NL SE**

(56) Entgegenhaltungen:
**FR-A-2 456 506**
**US-A-4 335 715**

(73) Patentinhaber: **Jokio, Pekka Johannes, Pihlajatie 45 A 5, SF- 00270 Helsinki (FI)**
Patentinhaber: **Lindholm, Thor Sam, Sveavägen 9 B Djursholm, S-182 64 Stockholm (SE)**

(72) Erfinder: **Jokio, Pekka Johannes, Pihlajatie 45 A 5, SF- 00270 Helsinki (FI)**
Erfinder: **Lindholm, Thor Sam, Sveavägen 9 B Djursholm, S-182 64 Stockholm (SE)**

(74) Vertreter: **Weitzel, Wolfgang, Dr.- Ing., St. Pöltener Strasse 43, D-7920 Heidenheim (DE)**

## Beschreibung

Den Gegenstand dieser Erfindung bildet ein Passer für Schienbeinoperationen, die zur Korrigierung von fehlerhaften Stellungen des Knies ausgeführt werden, welcher Passer zwei oder mehrere, in einem gewünschten Winkel zueinander in das Schienbein zu bohrende Nadeln, ein zumindest zwei Nadeln verbindendes, mit einer Gradskala versehenes lösbares und an einem Ende eine Führung für eine Nadel (6) aufweisendes Körperelement, und einen am Körperelement beweglichen und an ihm arretierbaren und eine Führung für eine andere Nadel (7) aufweisendes Arretierteil umfasst.

Ein Passer dieser Gattung ist aus US-A-4 335 715 bekannt.

Krankheiten, bei denen am Knie mit der Zeit ein fehlerhafter Winkel entsteht, d. h. die durch das Schenkelbein und das Schienbein gebildete sog. mechanische Achse des Beines verläuft nicht durch die Mitte des Knies, sind recht allgemein besonders bei alten Leuten, aber können auch bei jüngeren vorkommen. Die Ursache der Krankheit ist nicht mit Sicherheit bekannt. Einige möglichkeiten sind Brüche an Gelenkflächen des Knies und Gelenkscheibenschädigungen. Als äusseres Kennzeichen ist entweder O-Beinigkeit oder X-Beinigkeit.

Verfall im mittleren Teil des Knies führt zur O-Beinigkeit, wobei auf die Gelenkflächen eine ungleichmässige und mit der normalen Belastung verglichen stellenweise erheblich grössere Belastung grichtet wird. Zum Beispiel eine Formänderung von ca. 10 Grad führt zur Verdreifachung der Belastungskräfte. Dabei werden die an den Seiten des Knies befindlichen Gelenkbänder gedehnt, und die Krankheit schreitet immer schneller fort.

Als Behandlung für die obenbeschriebene Krankheit hat man u. a. Physiotherapie versucht, aber der von ihr gebrachte Nutzen ist ungenügend oder höchstens vorübergehend. Es hat sich nämmlich herausgestellt, dass wenn der fehlerhafte Winkel des Knies nicht ganz korrigiert werden kann, verschlimmert sich die Krankheit schnell wieder. Als beste Behandlung hat sich eine hohe Tibiaosteotomie, d. h. hohe Durchtrennung des Schienbeines, erwiesen. In ihr wird aus dem oberen Teil des Schienbeines nahe dem Knie ein keilförmiges Stück entfernt, wodurch der fehlerhafte Winkel des Knies ganz verschwindet.

Ver der Operation ist natürlich die Grösse des fehlerhaften Winkels des Knies möglichst genau zu bestimmen, um den Spitzwinkel des aus dem Schienbein zu entfernenden Keiles festzustellen. Dies geschieht am besten so, dass der Winkel aus einer Röntgenaufnahme abgemessen wird. Dabei wird in der Mitte des Schienbeines eine längsgerichtete, von oben nach unter reichende Achse gezeichnet, und für das Schenkelbein wird eine entsprechende Achse gezeichnet. Der Winkel zwischen diesen Achsen ist wesentlich derselbe wie der Spitzwinkel des zu entfernenden Keils - wenn man eine kleine Überkorrektion nicht machen will, die auch manchmal erforderlich ist.

Bei der Operation selbst ist es schwierig gewesen, einen Keil von genau richtiger Grösse zu entfernen. Verschiedene Hilfsmittel sind benutzt worden, aber bisher hat sich der von Lippert 1975 entwickelte Passer deutlich als das beste erwiesen. Er umfasst zwei sog. Kirschner-Nadeln sowie ein diese verbindendes, mit einer Gradskala versehenes Körperelement. Die Nadeln haben eine scharfe Spitze und werden im gewünschten Winkel zueinander durch den Knochen gebohrt. Danach, wenn der richtige Winkel markiert worden ist, wird das Entfernen des keilförmigen Stückes mit einer oszillierenden Säge ausgeführt.

Mit einem Passer dieser Art kann der zu korrigierende Winkel recht genau bestimmt und markiert werden, aber die Schneideebenen für den zu entfernenden Keil bleiben ausserhalb der Messung und sind nach Augenmass abzuschätzen. Es ist jedoch ebenso wichtig, das Schneiden auf einer richtigen, im voraus bestimmten Ebene durchzuführen.

Zweck dieser Erfindung ist, einen Passer, der obenbeschriebenen Gattung zu bilden, mit dem der durch eine hohe Tibiaosteotomie-Operation zu korrigierende Winkel abgemessen und die Ebene, auf der der Keil zu entfernen ist, markiert wird, oder in der Bogen-Osteotomie den Winkel der angestrebten Korrektion und die Ebene, auf der die Korrektion ausgeführt werden sollte, zu markieren. Ein Passer nach der Erfindung ist dadurch gekennzeichnet, dass zur Markierung der Schneideebenen des aus dem Schienbein auszuschneidenden Knochenkeils der Passer mit an den Nadeln montierbaren, am Knochen anbringbaren Schneideebene-Markierern ausgerüstet ist.

Eine günstige Anwendungsform der Erfindung ist dadurch gekennzeichnet, dass es der Schneideebene-Markierer zwei sind, eine für jede der beiden Nadeln.

Eine andere günstige Anwendungsform der Erfindung ist dadurch gekennzeichnet, dass zumindest die inneren, einander gegenüberliegenden Flächen der Schneideebene-Markierer wesentlich planar ausgebildet sind.

Eine günstige Anwendungsform der Erfindung ist noch dadurch gekennzeichnet, dass die Seite der Schneideebene-Markierer, die am Knochen angebracht wird, mit einer oder mehreren scharfen Befestigungsspiten versehen ist.

Als grösste Vorteile der Erfindung können erwähnt werden, dass sie leicht und einfach zu benutzen ist und dass sie es gewährleistet, dass der fehlerhafte Winkel in jedem Arthrosefall durch die Operation genau korrigiert wird und dass die Schneideebenen genau die richtigen sind und Abschätzungen nach Augenmass nicht erforderlich sind. Ausserdem ist es möglich, mit Hilfe der Schneideebene-Markierer eventuelle Ausstreckungsdefizite des Knies zu korrigieren.

Die Erfindung wird im folgenden mit Anwendungsformbeispielen erläutert, wobei auf die beigelegten Zeichnungen hingewiesen wird, wo

Fig. 1 ein Schienbein und ein Schenkelbein darstellt, dessen gegenseitiger Winkel fehlerhaft ist, sowie einen Passer, der bei der zur Korrigierung des Fehlers durchzuführenden Operation benutzt wird.

Fig. 2 dasselbe darstellt wie Fig. 1, aber jetzt sind am Passer erfindungsgemässe Schneideebene-Markierer montiert.

Fig. 3 einen der Erfindung entsprechenden, mit Schneideebene-Markierern ausgerüsteten Passer perspektivisch darstellt.

Fig. 4 dasselbe darstellt wie Fig. 3, aber jetzt sind vier Nadeln montiert.

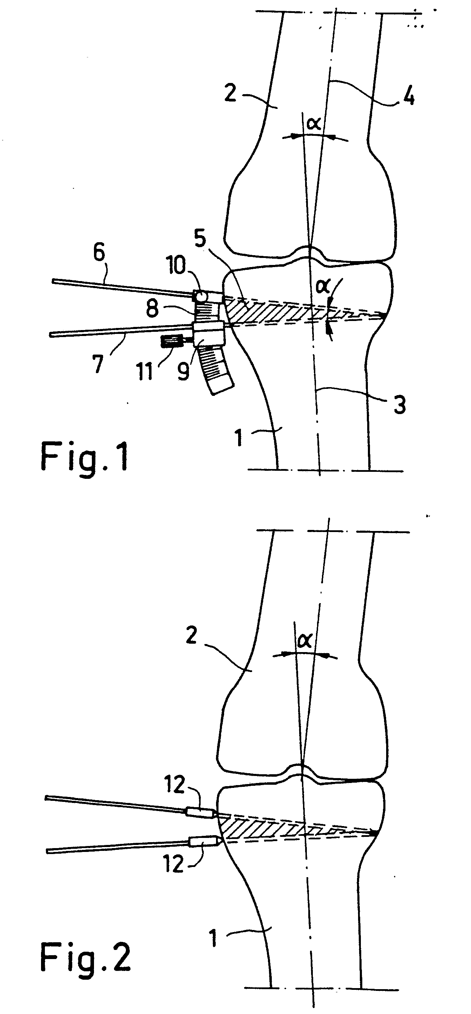

In Figur 1 ist die Benutzung des Passers bei der betreffenden Operation schematisch dargestellt. Wie man sieht, ist der Winkel des Knies fehlerhaft. In der Längsrichtung des Schienbeines 1 ist eine Achse 3 gezeichnet. In der Längsrichtung auch des Schenkelbeines 2 ist eine Achse 4 gezeichnet. Der Winkel $\alpha$ zwischen den Achsen 3 und 4 bestimmt den Winkel des aus dem Schienbein durch die Operation zu entfernenden keilförmigen Stückes 5, welcher Winkel ebenso gross ist wie der erstgenannte.

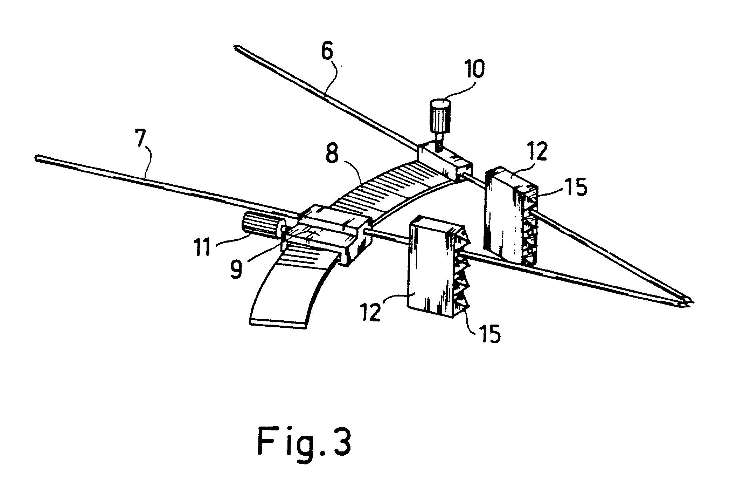

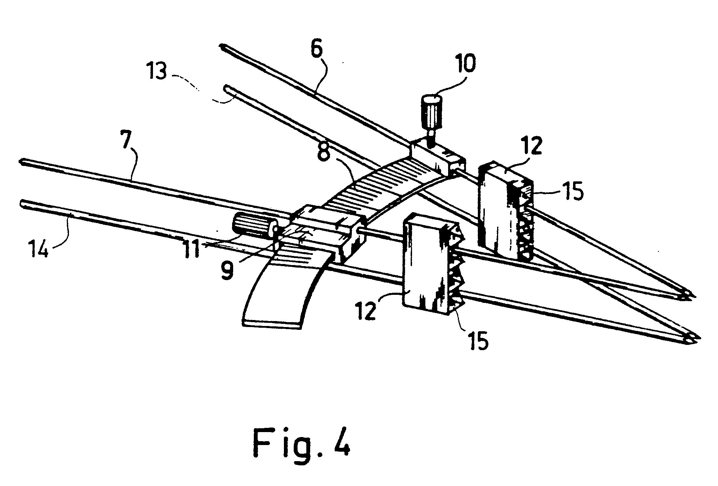

Ein der Erfindung entsprechender Passer, der bei der Operation als Hilfsmittel benutzt wird, ist in Figuren 3 und 4 detailliert dargestellt. Zum Passer gehören zwei oder mehrere, im allgemeinen aber eine gerade Zahl scharfspitziger Nadeln 6, 7, 13, 14, die in der Fachsprache als Kirschner-Nadeln bekannt sind. Von diesen Nadeln sind zumindest zwei 6, 7 durch ein mit einer Gradskala versehenes Körperelement 8 miteinander verbunden. An einem Ende des Körperelements ist eine Bohrung oder ein Einschnitt gebildet, wurch welche bzw. welchen die Nadel 6 geschoben ist. Die Nadel kann in ihrer Stellung arretiert werden durch Anziehen einer Schraube 10. Weiter gehört zum Passer ein Arretierteil 9, der am Körperelement 8 beweglich ausgeführt ist. Im Arretierteil 9 ist eine Bohrung für die andere Nadel 7. Der Arretierteil kann am Körperelement mit einer Schraube 11 arretiert werden. Ausserdem gehören zum Passer zwei Schneideebene-Markierer 12, einer für jede der beiden Nadeln 6, 7. An der Vorderseite des Schneideebene-Markierers - d. h. an der Seite, die gegen den Knochen kommt - sind scharfe Befestigungsspitzen 15 gebildet, die den Schneideebene-Markierer am Knochen halten. Wie viele Befestigunsspitzen da sind, ist nicht wesentlich. Die Hauptsache ist, der Schneideebene-Markierer bleibt fest am Knochen. Die Schneideebene-Markierer sind der Form nach schmale rechtwinklige Prismen. Sie können am günstigsten aus Metall oder hartem Kunststoff sein. Wesentich ist, dass sie steif sind. Es ist günstig, die grösseren Flächen der Schneideebene-Markierer planar zu bilden.

In Figuren 3 und 4 is auch zu sehen, dass in den Schneideebene-Markierern jeweils drei durchgehende Bohrungen für die Nadeln 6, 7, 13, 14 gebildet sind. Die Zahl der Bohrungen ist an sich willkürlich, aber es ist günstig, dass es ihrer wenigstens zwei sind und dass sie miteinander parall verlaufen und in der Seitenrichtung an derselben Stelle sind.

Die Benutzung der Schneideebene-Markierer als Hilfsmittel der Operation wird im folgenden Beispiel mit Hilfe der Figuren 1 und 2 erläutert. Wie früher erwähnt wurde, wird der fehlerhafte Winkel $\alpha$ des Knies am günstigsten aus einer Röntgenaufnahme ermittelt. Danach wird eine Kirschner-Nadel 6 durch das Schienbein 1 so gebohrt, dass sie an der hinteren Seite des Schienbeins ca. 5 mm herauskommt. Die Winkelskala des Körperelements ist auf eine bestimmte Entfernung von den Spitzen der Nadeln kalibriert, im allgemeinen 13,4 cm. Das Körperelement 8 ist während der Bohrung mit der Nadel 6 verbunden, oder kann wahlweise sofort danach montiert werden, wenn die Nadel 6 gebohrt worden ist. Der im Verhältnis zum Körperelement 8 bewegliche Arretierteil 9 wird auf die aus der Röntgenaufnahme ermittelte Gradzahl $\alpha$ gestellt und in dieser Stellung arretiert. Danach wird die andere Kirschner-Nadel 7 durch die im Arretierteil 9 befindliche Bohrung geschoben und in den Knochen gebohrt. Somit sind beide Nadeln 6, 7 im gewünschten Winkel $\alpha$ zueinander in das Schienbein 1 montiert. Danach werden das Körperelement 8 und der Arretierteil 9 abgenommen, so dass nur die Nadeln 6, 7 übrig bleiben.

Dadurch ist der Winkel des zu entfernenden Knochenkeiles bestimmt worden. Zur Markierung der Schneideebenen des Keiles wird an jede der beiden Nadeln ein Schneideebene-Markierer 12, der oben beschrieben wurde, geschoben. Die Schneideebene-Markierer werden mit dem Ende voran, das mit den Befestigungsspitzen 15 versehen ist, geschoben. Die Schneideebene-Markierer werden in eine der gewünschten Schneideebene entsprechende Stellung gestellt und mit den Befestigungsspitzen am Knochen angebracht. Danach werden die in Figur 4 dargestellten Nadeln 13, 14 durch die in den Schneideebene-Markierern befindlichen Bohrungen in das Schienbein geschoben auf dieselbe Weise wie die Nadeln 6, 7. Damit sind die Nadeln in gewünschten, die Schneideebene bestimmenden Stellungen arretiert worden, wonach die Schneideebene-Markierer 12 abgenommen werden können, und die Operation wird entlang den von den Nadeln 6, 13; 7, 14 bestimmten Ebenen ausgeführt, indem die Nadeln als Führungen benutzt werden. Die Operation erfolgt mit einer oszillierenden Säge.

Einem Fachmann der Branche ist es klar, dass die Erfindung nicht auf die obenangeführten Anwendungsformbeispiele beschränkt ist, sondern kann im Rahmen der nachstehenden Patentansprüche variiert werden. Somit kann z. B. das Körperelement 8 auf verschiedene Entfernungen gestellt werden, und die Höhe und Länge der Schneideebene-Markiererplatten 12 und ihre Befestigung geändert werden.

## Patentansprüche

1. Passer für Schienbeinoperationen, die zur Korrigierung von fehlerhaften Stellungen des Knies ausgeführt werden, welcher Passer zwei oder mehrere, in einem gewünschten Winkel zueinander in das Schienbein (1) zu bohrende Nadeln (6, 7, 13, 14), ein zumindest zwei Nadeln (6, 7) verbindendes, mit einer Gradskala versehenes lösbares und an einem Ende eine Führung für eine Nadel (6) aufweisendes Körperelement (8) und einen am Körperelement beweglichen und an ihm arretierbaren und eine Führung für eine andere Nadel (7) aufweisendes Arretierteil (9) umfasst, dadurch gekennzeichnet, dass zur Markierung der Schneideebenen des aus dem Schienbein (1) auszuschneidenden Knochenkeils der Passer mit an den Nadeln (6, 7) montierbaren, am Knochen anbringbaren Schneideebene-Markierern (12) ausgerüstet ist.

2. Passer nach Anspruch 1, dadurch gekennzeichnet, dass es der Schneideebene-Markierer (12) zwei sind, eine für jede der beiden Nadeln (6, 7).

3. Passer nach Anspruch 1 oder 2, dadurch gekennzeichnet, dass zumindest die inneren, einander gegenüberliegenden Flächen der Scheideebene-Markierer (12) wesentlich planar gestaltet sind.

4. Passer nach einem der Patentansprüche 1 - 3, dadurch gekennzeichnet, dass die Seite der Schneideebene-Markierer (12), die am Knochen (1) angebracht wird, mit einer oder mehreren scharfen Befestigungsspitzen (15) versehen ist.

5. Passer nach einem der Patentansprüche 1 - 4, dadurch gekennzeichnet, dass in jedem der beiden Schneideebene-Markierer (12) zumindest eine mit der genannten planaren Fläche parallel verlaufende, die Nadel (6, 7) durchlassende, sich durch den Schneideebene-Markierer hindurch erstreckende Bohrung gebildet ist.

6. Passer nach Patentanspruch 5, dadurch gekennzeichnet, dass in jedem der beiden Schneideebene-Markierer (12) zwei oder drei miteinander parallel verlaufende und in der Richtung der Normale der genannten planaren Fläche an wesentlich derselben Stelle befindliche, sich durch den Schneideebene-Markierer hindurch erstreckende Bohrungen gebildet sind.

## Claims

1. Adjuster for tibial operations performed to correct faulty positioning of the knee, which adjuster comprises two or more pins (6, 7, 13, 14) which are to be drilled at a desired angle to one another into the tibia (1), a detachable body element (8) which connects at least two pins (6, 7), is provided with a graduated scale and has at one end a guide for one pin (6), and a locking part (9) which can be moved on the body element and can be locked on it and has a guide for another needle (7), characterized in that in order to mark the cutting planes of the bone which is to be cut out of the tibia (1), the adjuster is equipped with cutting-plane markers (12) which can be mounted on the pins (6, 7) and can be brought into contact with the bone.

2. Adjuster according to Claim 1, characterized in that there are two of the cutting-plane markers (12), one for each of the two pins (6, 7).

3. Adjuster according to Claim 1 or 2, characterized in that at least the inner surfaces, which are opposite to one another, of the cutting-plane markers (12) are designed essentially planar.

4. Adjuster according to one of Patent Claims 1 - 3, characterized in that the side of the cutting-plane markers (12) which is brought into contact with the bone (1) is provided with one or more sharp fastening points (15).

5. Adjuster according to one of Patent Claims 1 - 4, characterized in that in each of the two cutting-plane markers (12) at least one hole is drilled right through the cutting-plane marker running parallel to the said planar surface and allowing the pins (6, 7) through.

6. Adjuster according to Patent Claim 5, characterized in that in each of the two cutting-plane markers (12) two or three holes are drilled right through the cutting-plane marker running parallel to one another and at essentially the same position in the direction of the normals to the said planar surface.

## Revendications

1. Gabarit pour opérations du tibia, qui est utilisé pour corriger des positions défectueuses du genou, ce gabarit comprend deux ou plusieurs aiguilles (6, 7, 13, 14) à introduire par perçage dans le tibia (1), qui font entre elles un angle souhaité, un élément de corps (8) démontable muni d'une échelle graduée et présentant à une extrémité, un guidage pour une aiguille (6) et une pièce de blocage mobile sur l'élément de corps et pouvant être bloquée sur lui et présentant un guidage pour une autre aiguille, caractérisé en ce que pour marquer les plans de coupe du coin d'os à enlever du tibia (1) le gabarit est équipé de marqueurs (12) de plans de coupe applicables sur l'os, montables sur les aiguilles

2. Gabarit selon la revendication 1, caractérisé en ce qu'il y a deux marqueurs de plan de coupe, un pour chacune des deux aiguilles (6, 7).

3. Gabarit selon la revendication 1 ou 2, caractérisé en ce qu'au moins les surfaces internes, se faisant face, des marqueurs de plan de coupe (12) sont essentiellement planes.

4. Gabarit selon l'une des revendications 1 à 3, caractérisé en ce que la face des marqueurs (12) de plan de coupe qui est appliquée sur l'os (1) est munie d'une ou plusieurs pointes de fixation (15) aiguisées.

5. Gabarit selon l'une des revendications 1 à 4, caractérisé en ce que dans chacun des deux marqueurs de plans de coupe (12) est formé au moins un trou s'étendant parallèlement aux surfaces planes citées traversant le marqueur de plan de coupe pour laisser passer les aiguilles (6, 7).

6. Gabarit selon la revendication 5, caractérisé en ce que dans chacun des deux marqueurs (12) de plan de coupe, sont formés deux ou trois trous s'étendant parallèlement entre eux et aux surfaces planes citées, se trouvant pour l'essentiel aux mêmes places, traversant totalement le marqueur de plan de coupe.

Fig.1

Fig.2

Fig.3

Fig. 4